# EUROPEAN PATENT SPECIFICATION

(11) **EP 0 674 506 B1**
(45) Date of publication and mention of the grant of the patent: **23.08.2000**
(21) Application number: 94902475.6
(22) Date of filing: 02.12.1993
(51) Int. Cl.: A61K 9/16, A61K 38/00

(54) **CONTROLLED RELEASE GROWTH HORMONE CONTAINING MICROSPHERES**
WACHSTUMHORMON ENTHALTENDE MIKROSPHAEREN MIT KONTROLLIERTER FREISETZUNG
MICROSPHERES CONTENANT UNE HORMONE DE CROISSANCE A LIBERATION REGULEE

(30) Priority: 02.12.1992 US 984323
(43) Date of publication of application: 04.10.1995
(60) Divisional application: 00101570.0
(73) Proprietor: Alkermes Controlled Therapeutics, Inc., Cambridge, MA 02139 (US)
(72) Inventor: AUER, Henry, Watertown, MA 02172 (US); KHAN, M. Amin, Burlington, MA 01803 (US); BERNSTEIN, Howard, Cambridge, MA 02139 (US)
(74) Representative: Kirkham, Nicholas Andrew
(86) International application number: PCT/US93/11621
(87) International publication number: WO 94/12158

(56) References cited:
- EP-A- 0 330 180
- WO-A-90/13780
- WO-A-93/17668

## Description

### Background of the Invention

This invention generally relates to polymeric microspheres for controlled release of growth hormone.

Human Growth hormone (hGH) is a protein secreted by the pituitary gland. The protein contains 191 amino acids and has a molecular weight of 22,000. hGH causes growth of all tissues of the body which are capable of growth. In addition, to its general effect in causing growth, the hormone is involved in the following metabolic effects: 1) increased cellular protein synthesis, 2) decreased glucose utilization, and 3) increased mobilization of fatty acids from adipose tissue. hGH is used to treat hypopituitary dwarfism and is administered three times per week by the subcutaneous or intramuscular route.

The administration of growth hormone generally necessitates frequent intramuscular (IM) or subcutaneous (SQ) injections. The advantages of a controlled release formulation for growth hormone include increased patient compliance and acceptance by reducing the number of injections, increased therapeutic benefit by eliminating the peak and valley changes in blood levels, and potentially lowering the total administered amount of drug by reducing peaks and valleys.

One means for controlling blood levels of a compound is to administer it in the form of a polymeric matrix that releases compound as a function of polymer degradation and/or drug diffusion. A variety of biodegradable and non-biodegradable polymers have been used for such applications, including polyesters such as poly(lactide-co-glycolide)s, polyanhydrides, polyorthoesters, and ethylenevinyl acetate polymers. In general, release is controlled by selection of the appropriate polymer, encapsulation conditions, and drug loading and excipients.

Examples of these polymeric systems are described in U.S. Patent Nos. 4,891,225 to Langer and 4,906,474 to Langer (polyanhydrides), 4,391,797 to Folkman, et al., (ethylenevinyl acetate polymers), 4,767,628 to Hutchinson (polylactide, polylactide-co-glycolide acid), and 4,530,840 to Tice, et al. (polylactide, polyglycolide, and copolymers).

EP-A-0330180 discloses microspheres which may comprise growth hormones and usual pharmaceutically acceptable excipients.

However, controlled release at the desired rate and over the desired period is difficult to achieve. Moreover, the conditions used to encapsulate the drug must not result in degradation of the drug to be delivered nor must the drug react with the polymeric matrix so as to inactivate or bind the drug. As important in a clinical situation, the delivery means must be cost effective to produce, stable to storage, and administrable using standard methodology.

It is therefore an object of the present invention to provide a method for making microspheres containing growth hormone with very little loss of activity or material, especially human growth hormone.

It is a further object of the present invention to provide a method for making microspheres formed from a broad range of polymers which contain active growth hormone releasable in a controlled fashion, and the microspheres produced by such a process.

### Summary of the Invention

Growth hormone polymeric controlled release systems are described wherein the growth hormone retains good biological activity and is released over an extended period of time following administration. In the preferred embodiment, the growth hormone polymeric microspheres are made using very cold temperatures to freeze the polymer-growth hormone mixtures into polymeric microspheres with very high retention of biological activity and material. Polymer, preferably a poly(lactide), is dissolved in a solvent such as methylene chloride together with powdered growth hormone. The polymer/growth hormone mixture is atomized into a vessel containing a frozen non-solvent such as ethanol, overlayed with a liquified gas such as nitrogen, at a temperature below the freezing point of the polymer/active agent solution or suspension. The atomized particles freeze into microspheres upon contacting the cold liquified gas, then sink onto the frozen non-solvent layer. The frozen non-solvent is then thawed. As the non-solvent thaws, the microspheres are still frozen and sink into the liquid non-solvent. The solvent in the microspheres also thaws and is slowly extracted into the non-solvent, resulting in hardened microspheres containing the growth hormone.

Examples using human growth hormone show sustained release of biologically active growth hormone when the microspheres are tested *in vitro* or *in vivo*, extending over a period of one day up to three months. Altered release can be achieved by inclusion of polymer degradation modifiers, pore forming agents, and stabilizers of the growth hormone.

### Brief Description of the Drawings

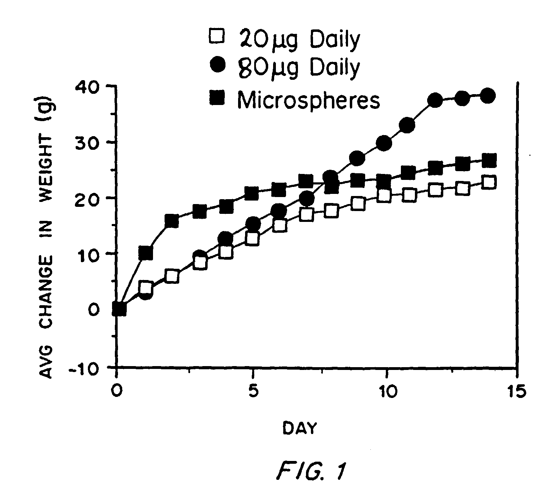

Figure 1 is a graph of the average change in weight (g) over 14 days for rats administered 80 µg growth hormone daily (dark circles), 20 µg growth hormone daily (open squares), and growth hormone released from microspheres (dark squares) over time (days).

### Detailed Description of the Invention

The invention is as defined in the claims. Growth hormone containing microspheres are made by incorporating the growth hormone into a biocompatible polymeric microsphere, up to approximately 50% w/w, wherein the microsphere containing the growth hormone is characterized by sustained controlled release of the growth hormone over a period of at least 24 hours up to a period of one to three months. In the preferred embodiment, the polymer is biodegradable, most preferably by hydrolysis, the microspheres have a diameter of less than 180 µm (microns), most preferably less than 70 µm (microns), and are suitable for administration by injection subcutaneously or intramuscularly (a size suitable for injection through a 23-gauge needle would be less than 180 µm in diameter), and the microspheres contain from 0.01% by weight up to approximately 50% by weight human growth hormone.

As used herein, "microsphere" is used to mean solid spheres formed of polymer having growth hormone dispersed throughout, as well as microparticulates and microcapsules, unless otherwise noted. Microparticulates are specifically referred to when describing irregularly shaped polymer or polymer-drug particles. Microcapsules are spherical shaped polymer devices having a non-polymer core or a core of a different polymer than the outer shell.

As used herein, "sustained" or "extended" release of the growth hormone can be continuous or discontinuous, linear or non-linear. This can be accomplished using one or more types of polymer compositions, drug loadings, selections of excipients or degradation enhancers, or other modifications, administered alone, in combination or sequentially to produce the desired effect.

Growth hormone is available in the form of a lyophilized powder containing physiological buffers and salts.

### Methods for incorporation of Growth hormone into microspheres.

A variety of techniques are known by which active agents can be incorporated into polymeric microspheres.

### Spray Drying

In spray drying, the polymer and growth hormone are mixed together in a solvent for the polymer, then the solvent is evaporated by spraying the solution, leaving polymeric droplets containing the active agent. Spray drying is reviewed in detail by K. Masters in "Spray Drying Handbook" (John Wiley & Sons, New York 1984); and Patrick B. Deasy in "Microencapsulation and Related Drug Processes" (Marcel Dekker, Inc., New York 1984). Spray drying is not preferred since it may result in some loss of activity due to the heat generated in the process as well as in loss of considerable amounts of the material due to sticking of the polymer to the large surface area on the sides of the chamber.

### Solvent Evaporation

Solvent evaporation techniques can be used to form microspheres. These techniques involve dissolving the polymer in an organic solvent which contains either dissolved or dispersed active agent. The polymer/active agent solution is then added to an agitated continuous phase which is usually aqueous. Emulsifiers are included in the aqueous phase to stabilize the oil-in-water emulsion. The organic solvent is then evaporated over a period of several hours or more, thereby depositing the polymer around the core material. Solvent can be removed from the microspheres in a single step, as described in U.S. Patent No. 3,737,337 and U.S. Patent No. 3,523,906, or in U.S. Patent No. 3,691,090 (under reduced pressure), or by the application of heat, as shown in U.S. Patent No. 3,891,570. A two-step technique is described in U.S. Patent No. 4,389,330. Freeze drying has also been used to remove the solvent from microspheres, as reported by Sato, et al, in "Porous Biodegradable Microspheres for Controlled Drug Delivery. I. Assessment of Processing Conditions and Solvent Removal Techniques," Pharmaceutical Research 5, 21-30 (1988).

Solvent evaporation works reasonably well but is not preferred since the amount of incorporated material is usually lower than the theoretical values due to loss of drug to the aqueous phase, as reported by Benita, et al., in "Characterization of Drug Loaded Poly(d,1-lactide) Microspheres," J. Pharm. Sci. 73, 1721-1724 (1984).

### Phase separation

Phase separation techniques can also be used to form microspheres. These techniques involve the formation of a water-in-oil emulsion or oil in water emulsion. The polymer is precipitated from the continuous phase onto the active agent by a change in temperature, pH, ionic strength or the addition of precipitants. For example, U.S. Patent No. 4,675,800, et al., describes the formation of poly(lactic-co-glycolic) acid microspheres containing active proteins. The protein is first dissolved in the aqueous phase of a water-in-oil emulsion or dispersed as a solid in the polymer phase. Polymer is then precipitated around the aqueous droplets or drug particles by addition of a non-solvent for the polymer such as silicone oil. The final product, as with most phase separation techniques, is in the form of a microcapsule. Microcapsules contain a core material surrounded by a polymer membrane capsule. Microcapsules are not the preferred embodiment for delivery of growth hormone, however, since the release kinetics of active agents from these devices can be difficult to control.

Although these phase separation techniques result in the formation of microspheres containing active agents, active agent is often lost during the solvent extraction process. In addition, as with spray drying, biologically active proteins may be denatured during the process.

### Rapid freezing, solvent extraction

The preferred method for making growth hormone microspheres having the desired characteristics is described in U.S. Patent No. 5,019,400 to Gombotz, et al.

There are two principal embodiments of the system for making microspheres: a combination liquified gas - frozen non-solvent system and a frozen non-solvent system.

Polymer and agent to be encapsulated in solution are atomized using an ultrasonic device into a liquified gas. The atomized particles freeze when they contact the liquified gas (liquid nitrogen), forming frozen spheres. These sink to the surface of the frozen non-solvent (ethanol). The liquid gas is evaporated and the spheres begin to sink into the non-solvent as the non-solvent thaws. The solvent in the spheres is extracted into the non-solvent to form microspheres containing the agent to be encapsulated. Other non-solvents such as hexane are added to the non-solvent (ethanol) to increase the rate of solvent extraction from certain polymers, where appropriate, for example, when spheres are formed of polylactide-co-glycolide polymers.

The liquified gas can be liquid argon (-185.6°C), liquid nitrogen (-195.8°C), liquid oxygen (-182.9°C) or any other gas that results in the immediate freezing of the atomized particles into frozen spheres. Oxygen is not preferred since it is explosive and may cause oxidation of the protein.

Alternatively, a cold non-solvent for the polymer can be substituted for the combination of liquified gas-frozen no-solvent, provided the temperature of the non-solvent is below the freezing temperature of the polymer/active agent solution.

In both embodiments, it is important that the polymer/active agent freeze immediately upon contacting the cold liquid, and then be slowly thawed and the polymer solvent extracted from the microspheres.

The thawing rate is dependent on the choice of solvents and non-solvents. It is important to select a solvent for the polymer having a higher melting point than the non-solvent for the polymer so that the non-solvent melts first, allowing the frozen microspheres to sink into the liquid where they later thaw. If a cold liquid non-solvent system for making the polymeric microspheres is used, the microspheres will sink immediately into the non-solvent. As the solvent in the microsphere thaws, it is extracted into the non-solvent. The solvent for the polymer and the non-solvent for the polymer must be miscible to allow extraction of the solvent from the microspheres. Table 1 shows some polymer/solvent/non-solvent systems that can be used in this process along with their melting points.

**Table 1**

| **Polymers and Appropriate Solvents and Non-Solvents Systems, with Solvent and Non-Solvent Melting Points** °**C** | | |
|---|---|---|
| POLYMER | SOLVENT | NON-SOLVENT |
| Poly(lactide | Methylene Chloride (-95.1) | Ethanol (-114.5) |
| | Chloroform (-63.5) | Methanol (-97.5) |
| Poly(lactide-co-glycolide acid) | Ethyl Acetate (-83.6) | Ethanol (-114.5) |
| | Acetone (-95.4) | Ethyl ether (-116.3) |
| | Methylene Chloride (-95.1) | Isopentane (-130) |
| Poly(caprolactone) | Methylene Chloride (-95.1) | Ethanol (-114.5) |
| Poly (vinyl alcohol) | Water (0) | Acetone (-95.4) |
| Ethylene-vinyl acetate | Methylene Chloride (-95.0) | Ethanol (-114.5) |

The polymer/active agent/solvent mixture can be sprayed into the cold liquid, either the liquified gas or the cold non-solvent, using a variety of devices which can be used to form small particles, including sonic nozzles, pressure nozzles, pneumatic nozzles and rotary atomizers.

A wide range of sizes of microspheres can be made by varying the droplet size, for example, by changing the nozzle diameter. If very large spheres are desired, the spheres can be extruded through a syringe directly into the cold liquid. Increasing the inherent viscosity of the polymer solution can also result in an increasing microspheres size. The size of the spheres produced by this process can range from greater than 1000 to 5 µm (microns) in diameter. A preferred size range for injectable microspheres is from 30 to 180 µm (microns) in diameter. The microspheres made by this technique are spherical in shape.

### Selection of the Polymeric Matrix

Polymers that can be used to form the microspheres include bioerodible polymers such as poly(lactide), poly(lactide-co-glycolide), poly(caprolactone), polycarbonates, polyamides, polyanhydrides, polyamino acids, polyortho esters, polyacetals, polycyanoacrylates and degradable polyurethanes, and non-erodible polymers such as polyacrylates, ethylene-vinyl acetate polymers and other acyl substituted cellulose acetates and derivatives thereof, non-erodible polyurethanes, polystyrenes, polyvinyl chloride, polyvinyl fluoride, poly(vinyl imidazole), chlorosulphonated polyolifins, and polyethylene oxide. Almost any type of polymer can be used provided the appropriate solvent and non-solvent are found which have the desired melting points. In general, a polymer solution is prepared containing between 1% polymer and 30% polymer, preferably 5-10% polymer.

In the preferred embodiment, a poly(lactide) is used. As used herein, this term includes polymers of lactic acid or lactide alone, copolymners of lactic acid and glycolic acid, copolymers of lactide and glycolide, mixtures of such polymers and copolymers, the lactic acid or lactide being either in racemic or optically pure form. It is most desirable to use polylactides in the range of molecular weight up to 100,000.

The release of the growth hormone from these polymeric systems can occur by two different mechanisms. The drug can be released by diffusion through aqueous filled channels generated in the dosage form by the dissolution of the drug or by voids created by the removal of the polymer solvent during the original microencapsulation. The second mechanism is enhanced release due to the degradation of the polymer. With time the polymer begins to erode and generates increased porosity and microstructure within the device. This creates additional pathways for drug release.

The degradation of the polymers occurs by spontaneous hydrolysis of the ester linkages on the backbone. Thus the rate can be controlled by changing polymer properties influencing water uptake. These include the monomer ratio (lactide to glycolide), the use of L-Lactide as opposed to D/L Lactide, and the polymer molecular weight. These factors determine the hydrophilicity and crystallinity which ultimately govern the rate of water penetration. Hydrophilic excipients such as salts, carbohydrates and surfactants can also be incorporated to increase water penetration into the devices and thus accelerate the erosion of the polymer.

By altering the properties of the polymer and the properties of the dosage form, one can control the contribution of each of these release mechanisms and alter the release rate of growth hormone. Slowly eroding polymers such as poly L-lactide or high molecular weight poly(lactide-co-glycolide) with low glycolide compositions will cause the release to become diffusion controlled. Increasing the glycolide composition and decreasing the molecular weight enhances both water uptake and the hydrolysis of the polymer and adds an erosion component to the release kinetics.

The release rate can also be controlled by varying the loading of growth hormone within the microspheres. Increasing the loading will increase the network of interconnecting channels formed upon the dissolution of the drug and enhance the release of drug from the microspheres. The preferred range of growth hormone loadings is in the range of 3-30% (w/w).

Polymer hydrolysis is accelerated at acidic or basic pH's and thus the inclusion of acidic or basic excipients can be used to modulate the polymer erosion rate. The excipients can be added as particulates, can be mixed with the incorporated growth hormone or can be dissolved within the polymer.

Excipients can be also added to the growth hormone to maintain its potency depending on the duration of release. Stabilizers include carbohydrates, amino acids, fatty acids, and surfactants and are known to those skilled in the art. In addition, excipients which modify the solubility of growth hormone such as salts, complexing agents (albumin, protamine) can be used to control the release rate of the protein from the microspheres.

### Additives to alter release rate, degradation rate, stability of growth hormone

Stabilizers for the growth hormone are based on ratio to the protein on a weight basis. Examples include carbohydrate such as sucrose, lactose, mannitol, dextran, and heparin, proteins such as albumin and protamine, amino acids such as arginine, glycine, and threonine, surfactants such as Tween™ and Pluronic™, salts such as calcium chloride and sodium phosphate, and lipids such as fatty acids, phospholipids, and bile salts.

The ratios are generally 1:10 to 4:1, carbohydrate to protein, amino acids to protein, protein stabilizer to protein, and salts to protein; 1:1000 to 1:20, surfactant to protein; and 1:20 to 4:1, lipids to protein.

Degradation enhancers are based on weight relative to the polymer weight. They can be added to the protein phase, added as a separate phase (i.e., as particulates) or can be codissolved in the polymer phase depending on the compound. In all cases the amount should be between 0.1 and thirty percent (w/w, polymer). Types of degradation enhancers include inorganic acids such as ammonium sulfate and ammonium chloride, organic acids such as citric acid, benzoic acids, heparin, and ascorbic acid, inorganic bases such as sodium carbonate, potassium carbonate, calcium carbonate, zinc carbonate, and zinc hydroxide, and organic bases such as protamine sulfate, spermine, choline, ethanolamine, diethanolamine, and triethanolamine and surfactants such as Tween™ and Pluronic™.

Pore forming agents to add microstructure to the matrices (i.e., water soluble compounds such as inorganic salts and sugars). They are added as particulates. The range should be between one and thirty percent (w/w, polymer).

### Administration of the microspheres to a patient.

An effective amount of the microspheres containing growth hormone are administered to a patient by injection subcutaneously, intramuscularly, intraperitoneally, and intradermally, by administration to mucosal membranes (such as intranasally or by means of a suppository), or by *in situ* delivery to provide the desired dosage of growth hormone, based on the known parameters for treatment with growth hormone of the various medical conditions.

The present invention is further described by the following examples.

### Example 1: Preparation of poly(L-lactic acid) microspheres containing growth hormone.

0.54 of poly D/L lactide co-glycolide 50:50 (Inherent viscosity 0.16) (Birmingham Polymers, Birmingham AL) was dissolved in 3.2 ml of methylene chloride. To this polymer solution was added 60 mg of lyophilized human growth hormone containing zinc ions at a 4:1 molar ratio and sodium bicarbonate (10 mg). The lyophilized protein had particle sizes in the 2-5 µm (micron) range. The solution was placed in 10 ml gas tight syringe. A 200 ml amount of 100% ethanol was added to round polypropylene container (17 cm diameter, 8 cm deep). This solution was frozen in liquid nitrogen and covered with 500 ml of liquid nitrogen. The polymer protein mixture was pumped from the syringe via a syringe pump at 2 ml/min, into an ultrasonic nozzle (Mode, Sonics and Material, Danbury CT) that was placed above the container of liquid nitrogen and frozen ethanol. The nozzle atomized the suspension into droplets which froze upon contact with the liquid nitrogen and formed microspheres which sank onto the frozen ethanol.

The container was placed at -80°C where the liquid nitrogen evaporated and the ethanol melted with time. As the ethanol thaws, the microspheres settle into the liquid where the methylene chloride is extracted. After 24 hours, an additional 200 ml of 100% ethanol prechilled to -80°C was added to the container. After three days, the slurry of microspheres and ethanol was filtered using a 1 µm (micron) Durapore membrane (Millipore, Bedford, MA). The filtered microspheres were then lyophilized.

### Example 2: In vivo assay of growth hormone released from poly(D/L-lactide co-glycolide) miorospheres.

Microspheres as produced in example 1 were tested in the rat bioassay detailed in the British Pharmacopeia. Hypophysectomized rats were obtained from Taconics, Germantown, New York. Three groups of animals were utilized. Groups 1 and 2 received a single daily injection of hGH of 20 and 80 µg respectively. Group 3 received 18 mg of microspheres injected subcutaneously in the dorsum. The animals were fed with a standard diet and allowed free access to water.

The increase in body weight is shown in Figure 1. The injectable formulation provides sustained release of potent hormone which results in body weight gain.

## Claims

1. A polymeric microsphere having a diameter of less than 1000 µM (microns), formed of a biocompatible polymer selected from the group consisting of poly(lactide), poly(lactide-co-glycolide)s, poly(caprolactone), polycarbonates, polyamides, polyanhydrides, polyamino acids, polyortho esters, polyacetals, polycyanoacrylates, degradable polyurethanes, polyacrylates polymers of ethylene-vinyl acetate and other acyl substituted cellulose acetates and derivatives thereof, polysaccharides, non-erodible polyurethanes, polystyrenes, polyvinyl chloride, polyvinyl fluoride, poly(vinyl imidazole), chlorosulphonated polyolefins, polyethylene oxide, copolymers and mixtures thereof, containing growth hormone dispersed through the polymer in a concentration of between 0.1% and 50% by weight, in combination with an excipient modifying growth hormone solubility present in a concentration of between 0.1 and thirty percent (w/w, polymer) which is selected from the group consisting of salts, complexing agents, inorganic acids, organic acids, inorganic bases, organic bases, and surfactants and an excipient modulating polymer erosion rate wherein the growth hormone is released under physiological conditions over a period of time greater than one day.

2. The microspheres of claim 1 wherein the erosion rate modulating agent is a pore forming agent added to the polymer in particulate form in a concentration of between one and thirty percent (w/w, polymer).

3. The microspheres of Claim 1 further comprising a stabilizer selected from the group consisting of carbohydrates, amino acids, proteins, lipids, salts fatty acids, and surfactants.

4. The microspheres according to any one of claims 1 to 3, wherein the microsphere has a diameter of less than 180 µM (microns).

5. A biocompatible polymeric microsphere for use in the administration of growth hormone and containing between 0.1 and 50% growth hormone and having a diameter of less than 180 µM (microns), formed of a biocompatible polymer selected from the group consisting of poly(lactide), poly(lactide-co-glycolide)s, poly(caprolactone), polycarbonates, polyamides, polyanhydrides, polyamino acids, polyortho esters, polyacetals, polycyanoacrylates, degradable polyurethanes, polyacrylates, polymers of ethylene-vinyl acetate and other acyl substituted cellulose acetates and derivatives thereof, polysaccharides, non-erodible polyurethanes, polystyrenes, polyvinyl chloride, polyvinyl fluoride, poly(vinyl imidazole), chlorosulphonated polyolefins, polyethylene oxide, copolymers and mixtures thereof, containing growth hormone dispersed through the polymer in a concentration of between 0.1% and 50% by weight and an excipient selected from the group consisting of excipients modulating polymer erosion rate, excipients stabilizing human growth hormone potency, and excipients modifying the solubility of growth hormone, wherein the growth hormone is released under physiological conditions over a period of time in excess of one day.

6. The biocompatible polymeric microsphere according to claim 5, wherein the microsphere is for use in the administration of growth hormone by injection intramuscularly, subcutaneously, intraperitoneally, intradermally or by application to the muscosal membrane.

7. The biocompatible polymeric microsphere according to claim 5 or claim 6, wherein the microsphere has a diameter of less than 70 µM.

8. A method for making a device for controlled sustained administration of growth hormone comprising
making polymeric microspheres having a diameter of less than one hundred and eighty microns, formed of a biocompatible polymer selected from the group consisting of poly(lactide), poly(lactide-co-glycolide), poly(caprolactone), polycarbonates, polyamides, polyanhydrides, polyamino acids, polyortho esters, polyacetals, polycyanoacrylates, degradable polyurethanes, polyacrylates, polymers of ethylene-vinyl acetate and other acyl subsituted cellulose acetates and derivates thereof, non-erodible polyurethanes, polystyrenes, polyvinyl chloride, polyvinyl fluoride, poly(vinyl imidazole), chlorosulphonated polyolefins, polyethylene oxide, and copolymers and mixtures thereof containing growth hormone in a concentration of between 0.1% and 50% by weight, wherein the microspheres further comprise an excipient modulating polymer erosion rate, and an excipient modifying the solubility of growth hormone by
a) freezing droplets of polymer-growth hormone solution by atomizing the droplets into a liquified gas, having a temperature below the freezing point of the polymer solution effective to immediately freeze the atomized polymer solution upon contact, said liquified gas overlaying a layer of frozen liquid non-solvent for the polymer, wherein the polymer solvent is miscible in the liquid non-solvent;
b) thawing the polymer solvent in the frozen droplets of polymer solution; and
c) extracting the solvent from the droplets into a liquid non-solvent to form spherical polymeric microspheres.

9. The method of claim 8 wherein (a) the erosion rate modulating agent is a pore forming agent added to the polymer in particulate form in a concentration of between one and thirty percent (w/w, polymer); or (b) the microsphere further comprises a stabilizer selected from the group consisting of carbohydrates, amino acids, proteins, lipids, salts, fatty acids, and surfactants; or (c) the excipients which modify the solubility of growth hormone are present in a concentration of between 0.1 and thirty percent (w/w, polymer) and are selected from the group consisting of salts, complexing agents, inorganic acids, organic acids, inorganic bases, organic bases, and surfactants.

10. The microspheres of any one of claims 1 to 7 for use in therapy.

11. Use of the microspheres of any one of claims 1 to 7 for the manufacture of a medicament for use in growth hormone therapy.

## Patentansprüche

1. Eine Polymermikrosphäre mit einem Durchmesser von weniger als 1000 µm (Mikron), die aus einem biokompatiblen Polymer gebildet ist, das aus der Gruppe ausgewählt ist bestehend aus Poly(lactid), Poly(lactid-co-glycolid)en, Poly(caprolacton), Polycarbonaten, Polyamiden, Polyanhydriden, Polyaminosäuren, Polyorthoestern, Polyacetalen, Polycyanoacrylaten, abbaubaren Polyurethanen, Polyacrylaten, Ethylenvinylacetat-Polymeren und anderen acyl-substituierten Celluloseacetaten und Derivaten davon, Polysacchariden, nicht-erodierbaren Polyurethanen, Polystyrolen, Polyvinylchlorid, Polyvinylfluorid, Poly(vinylimidazol), chlorsulfonierten Polyolefinen, Polyethylenoxid, Copolymeren und Mischungen davon, wobei die Polymermikrosphäre ein Wachstumshormon, das im Polymer in einer Konzentration zwischen 0,1 und 50 Gew.-% dispergiert ist, zusammen mit einem die Löslichkeit des Wachstumshormons modifizierenden Arzneimittelträger, der in einer Konzentration zwischen 0,1 und dreißig Prozent (w/w, Polymer) vorhanden ist und aus der Gruppe ausgewählt ist bestehend aus Salzen, Komplexbildnern, anorganischen Säuren, organischen Säuren, anorganischen Basen, organischen Basen und oberflächenaktiven Stoffen, und einem die Polymer-Erosionsrate modulierenden Arzneimittelträger enthält, worin das Wachstumshormon unter physiologischen Bedingungen über einen Zeitraum von mehr als einem Tag freigesetzt wird.

2. Die Mikrosphären nach Anspruch 1, worin das die Erosionsrate modulierende Mittel ein Poren-bildendes Mittel ist, das zu dem Polymer in partikulärer Form in einer Konzentration zwischen einem und dreißig Prozent (w/w, Polymer) gegeben ist.

3. Die Mikrosphären nach Anspruch 1, die außerdem einen Stabilisator umfassen, der aus der Gruppe ausgewählt ist bestehend aus Kohlenhydraten, Aminosäuren, Proteinen, Lipiden, Salzen, Fettsäuren und oberflächenaktiven Stoffen.

4. Die Mikrosphären gemäß einem der Ansprüche 1 bis 3, worin die Mikrosphäre einen Durchmesser von weniger als 180 µm (Mikron) besitzt.

5. Eine biokompatible Polymermikrosphäre zur Verwendung bei der Verabreichung von Wachstumshormon, wobei die Polymermikrosphäre zwischen 0,1 und 50% Wachstumshormon enthält, einen Durchmesser von weniger als 180 µm (Mikron) besitzt und aus einem biokompatiblen Polymer gebildet ist, das aus der Gruppe ausgewählt ist bestehend aus Poly(lactid), Poly(lactid-co-glycolid)en, Poly(caprolacton), Polycarbonaten, Polyamiden, Polyanhydriden, Polyaminosäuren, Polyorthoestern, Polyacetalen, Polycyanoacrylaten, abbaubaren Polyurethanen, Polyacrylaten, Ethylenvinylacetat-Polymeren und anderen acyl-substituierten Celluloseacetaten und Derivaten davon, Polysacchariden, nicht-erodierbaren Polyurethanen, Polystyrolen, Polyvinylchlorid, Polyvinylfluorid, Poly(vinylimidazol), chlorsulfonierten Polyolefinen, Polyethylenoxid, Copolymeren und Mischungen davon, wobei die Polymermikrosphäre ein Wachstumshormon, das im Polymer in einer Konzentration zwischen 0,1 und 50 Gew.-% dispergiert ist, und einen Arzneimittelträger enthält, der aus der aus Arzneimittelträgern bestehenden Gruppe ausgewählt ist, bestehend aus Arzneimittelträgern, die die Polymer-Erosionsrate modulieren, Arzneimittelträgern, die die Wirksamkeit des Wachstumshormons stabilisieren und Arzneimittelträgern, die die Löslichkeit des Wachstumshormons modifizieren, worin das Wachstumshormon unter physiologischen Bedingungen über einen Zeitraum von mehr als einem Tag freigesetzt wird.

6. Die biokompatible Polymermikrosphäre gemäß Anspruch 5, worin die Mikrosphäre zur Verwendung bei der Verabreichung von Wachstumshormon durch intramuskuläre, subcutane und intraperitoneale, intradermale Injektion oder durch Applikation auf die Mukosa ist.

7. Die biokompatible Polymermikrosphäre gemäß Anspruch 5 oder Anspruch 6, worin die Mikrosphäre einen Durchmesser von weniger als 70 µm besitzt.

8. Ein Verfahren zur Herstellung einer Vorrichtung zur kontrollierten Langzeit-Verabreichung von Wachstumshormon, wobei das Verfahren umfasst
Herstellen von Polymermikrosphären mit einem Durchmesser von weniger als einhundertachtzig Mikron, die aus einem biokompatiblen Polymer gebildet sind, das aus der Gruppe ausgewählt ist bestehend aus Poly(lactid), Poly(lactid-co-glycolid), Poly(caprolacton), Polycarbonaten, Polyamiden, Polyanhydriden, Polyaminosäuren, Polyorthoestern, Polyacetalen, Polycyanoacrylaten, abbaubaren Polyurethanen, Polyacrylaten, Ethylenvinylacetat-Polymeren und anderen acyl-substituierten Celluloseacetaten und Derivaten davon, nicht-erodierbaren Polyurethanen, Polystyrolen, Polyvinylchlorid, Polyvinylfluorid, Poly(vinylimidazol), chlorsulfonierten Polyolefinen, Polyethylenoxid und Copolymeren und Mischungen davon, wobei die Polymermikrosphären ein Wachstumshormon in einer Konzentration zwischen 0,1 und 50 Gew.-% enthalten, worin die Mikrosphären außerdem einen die Polymer-Erosionsrate modulierenden Arzneimittelträger und einen die Löslichkeit des Wachstumshormons modifizierenden Arzneimittelträger umfassen, durch
a) Gefrieren von Tröpfchen aus Polymer-Wachstumshormon Lösung durch Zerstäuben der Tröpfchen in ein Flüssiggas, das eine Temperatur unterhalb des Gefrierpunktes der Polymer-Lösung besitzt und wirksam ist, sofort bei Kontakt die zerstäubte Polymer-Lösung gefrieren zu lassen, wobei besagtes Flüssiggas eine Schicht gefrorenen flüssigen Nicht-Lösemittels für das Polymer überlagert, worin das Polymer-Lösemittel in dem flüssigen Nicht-Lösemittel mischbar ist;
b) Auftauen des Polymer-Lösemittels in den gefrorenen Tröpfchen aus Polymer-Lösung; und
c) Extrahieren des Lösemittels aus den Tröpfchen in ein flüssiges Nicht-Lösemittel, um sphärische Polymermikrosphären zu bilden.

9. Das verfahren nach Anspruch 8, worin (a) das die Erosionsrate modulierende Mittel ein Poren-bildendes Mittel ist, das zu dem Polymer in partikulärer Form in einer Konzentration zwischen einem und dreißig Prozent (w/w, Polymer) gegeben ist; oder (b) die Mikrosphäre außerdem einen Stabilisator umfasst, der aus der Gruppe ausgewählt ist bestehend aus Kohlenhydraten, Aminosäuren, Proteinen, Lipiden, Salzen, Fettsäuren und oberflächenaktiven Stoffen; oder (c) die Arzneimittelträger, die die Löslichkeit des Wachstumshormons modifizieren, in einer Konzentration zwischen 0,1 und dreißig Prozent (w/w, Polymer) vorhanden sind und aus der Gruppe ausgewählt sind bestehend aus Salzen, Komplexbildnern, anorganischen Säuren, organischen Säuren, anorganischen Basen, organischen Basen und oberflächenaktiven Stoffen.

10. Die Mikrosphären nach einem der Ansprüche 1 bis 7 zur therapeutischen Verwendung.

11. Verwendung der Mikrosphären nach einem der Ansprüche 1 bis 7 zur Herstellung eines Medikaments zur Verwendung in einer Therapie mit Wachstumshormonen.

## Revendications

1. Microsphère de polymère présentant un diamètre inférieur à 1000 µM (microns), composé d'un polymère biocompatible choisi dans le groupe constitué des poly(lactide), pol(lactide-co-glycolide)s, poly(caprolactone), polycarbonates, polyamides, polyanhydrides, poly(aminoacides), polyortho esters, polyacétals, polycyanoacrylates, polyuréthanes dégradables, polyacrylates, polymères de l'éthylène et de l'acétate de vinyle, et autres acétates de cellulose substitués par acyle et leurs dérivés, polysaccharides, polyuréthanes non-érodables, polystyrènes, poly(chlorure de vinyle), poly(fluorure de vinyle) poly(vinylimidazole), polyoléfines chlorosulfonées, poly(oxyde d'éthylène), copolymères et leurs mélanges, contenant de l'hormone de croissance dispersée dans le polymère à une concentration de 0,1 à 50% en poids, en combinaison avec un excipient capable de modifier la solubilité de l'hormone de croissance présent dans une concentration comprise entre 0,1 et 30% (poids/poids, polymère) qui est choisi dans le groupe constitué des sels, des agents complexants, des acides inorganiques, des acides organiques, des bases inorganiques, des bases organiques et des tensioactifs et un excipient capable de moduler le taux d'érosion du polymère, dans laquelle l'hormone de croissance est libérée dans des conditions physiologiques sur une période supérieure à un jour.

2. Microsphères selon la revendication 1, dans lesquelles l'agent capable de moduler le taux d'érosion est un agent formant pore ajouté au polymère en un état particulaire en une concentration comprise entre 1 et 30% (poids/poids, polymère).

3. Microsphères selon la revendication 1, contenant en outre un agent stabilisant choisi dans le groupe constitué des glucides, des aminoacides, des protéines, des lipides, des sels d'acides gras, et des tensioactifs.

4. Microsphères selon l'une quelconque des revendications 1 à 3, présentant un diamètre inférieur à 180 µM (microns).

5. Microsphères de polymère biocompatible utilisées en vue d'administrer une hormone de croissance et contenant entre 0,1 et 50% d'hormone de croissance et présentant un diamètre inférieur à 180 µM (microns) composées d'un polymère biocompatible choisi dans le groupe constitué des poly(lactide), poly(lactide-co-glycolide)s, polycaprolactone, polycarbonates, polyamides, polyanhydrides, polyaminoacides, poly-orthoesters, polyacétals, polycyanoacrylates, polyuréthanes dégradables, polyacrylates, polymères de l'éthylène et de l'acétate de vinyle et autres acétates de cellulose substitués par acyle et leurs dérivés, polysaccharides, polyuréthanes non érodables, polystyrènes, poly(chlorure de vinyle), poly(fluorure de vinyle), poly(vinylimidazole), polyoléfines chlorosulfonées, poly(oxyde d'éthylène), copolymères et leurs mélanges, contenant de l'hormone de croissance dispersée dans le polymère à une concentration comprise entre 0,1 et 50% en poids et un excipient choisi dans le groupe constitué des excipients capables de moduler le taux d'érosion du polymère, des excipients stabilisant l'activité de l'hormone de croissance humaine, et des excipients capables de modifier la solubilité de l'hormone de croissance, dans laquelle l'hormone de croissance est libérée dans des conditions physiologiques sur une période supérieure à un jour.

6. Microsphère de polymère biocompatible selon la revendication 5, dans laquelle la microsphère est utilisée en vue de l'administration d'une hormone de croissance par injection intramusculaire, sous-cutanée, intrapéritonéale, intradermique ou par application sur une membrane de muqueuse.

7. Microsphère de polymère biocompatible selon la revendication 5 ou la revendication 6, présentant un diamètre de 70 µM.

8. Procédé pour la production d'un dispositif pour l'administration continue et contrôlée d'une hormone de croissance, comprenant la production de microsphères de polymère présentant un diamètre inférieur à 180 µM, composées d'un polymère biocompatible choisi dans le groupe constitué des poly(lactide), pol(lactide-co-glycolide)s, poly(caprolactone), polycarbonates, polyamides, polyanhydrides, poly(aminoacides), polyortho-esters, polyacétals, polycyanoacrylates, polyuréthanes dégradables, polyacrylates, polymères de l'éthylène et de l'acétate de vinyle, et autres acétates de cellulose substitués par acyle et leurs dérivés, polysaccharides, polyuréthanes non-érodables, polystyrènes, poly(chlorure de vinyle), poly(fluorure de vinyle), poly(vinylimidazole), polyoléfines chlorosulfonées, poly(oxyde d'éthylène), copolymères et leurs mélanges, contenant de l'hormone de croissance dans une concentration comprise entre 0,1 et 50% en poids, dans laquelle les microsphères comprennent en outre un excipient capable de moduler le taux d'érosion du polymère et un excipient capable de modifier la solubilité de l'hormone de croissance par :
a) congélation de gouttelettes d'une solution polymère-hormone de croissance par atomisation des gouttelettes dans un gaz liquéfié présentant une température inférieure au point de congélation de la solution de polymère capable de provoquer la congélation immédiate de la solution de polymère atomisée par contact, ledit gaz liquéfié étant disposé dans une couche située au-dessus de la couche de liquide de congélation, qui est un liquide non-solvant vis-à-vis du polymère, le solvant du polymère étant miscible dans le liquide non-solvant ;
b) décongélation du solvant du polymère dans les gouttelettes congelées de la solution de polymère ; et
c) extraction du solvant des gouttelettes dans un liquide non-solvant de façon à former des microsphères de polymère sphériques.

9. Procédé selon la revendication 8, dans lequel :
a) l'agent de modulation du taux d'érosion est un agent formant pore ajouté au polymère en un état particulaire en une concentration comprise entre 1 et 30% (poids/poids, polymère) ; ou
b) la microsphère comprend en outre un agent stabilisant choisi dans le groupe constitué des glucides, aminoacides, protéines, lipides, sels, acides gras, et tensio-actifs ; ou
c) les excipients capables de modifier la solubilité de l'hormone de croissance sont présents en une concentration comprise entre 0,1 et 30% (poids/poids, polymère) et sont choisis dans le groupe constitué des sels, agents complexants, acides inorganiques, acides organiques, bases inorganiques, bases organiques, et tensio-actifs.

10. Microsphères selon l'une quelconque des revendications 1 à 7, destinées à une utilisation en thérapie.

11. Utilisation des microsphères selon l'une quelconque des revendications 1 à 7, pour la fabrication d'un médicament destiné à une thérapie à base d'hormones de croissance.
